# EUROPEAN PATENT SPECIFICATION

(11) **EP 0 493 819 B1**
(45) Date of publication and mention of the grant of the patent: **16.08.1995**
(21) Application number: 91122319.6
(22) Date of filing: 27.12.1991
(51) Int. Cl.: G01N 33/18

(54) **Water quality checker**
Wasserqualitätsprüfer
Dispositif de contrôle de la qualité de l'eau

(30) Priority: 30.12.1990 JP 416956/90
(43) Date of publication of application: 08.07.1992
(73) Proprietor: HORIBA, LTD., Minami-ku Kyoto (JP)
(72) Inventor: Mori, Takeshi, Kyoto (JP); Ohkawa, Hiromi, Joyo-city, Kyoto (JP); Kohno, Satoshi, Kyoto (JP)
(74) Representative: TER MEER STEINMEISTER & PARTNER GbR

(56) References cited:
- EP-A- 0 220 666
- EP-A- 0 417 571
- WO-A-85/01579
- WO-A-90/01160
- GB-A- 2 219 398

## Description

The invention relates to a water quality checker used for measuring the water quality for example of rivers or the like.

For a reliable judgement of the quality of water for example in rivers it is necessary to measure many items such as the pH, the dissolved oxygen, the conductivity and turbidity. Water quality checkers provided with a plurality of kinds of measuring sensors are known in the art or have been proposed for carrying out these respective measurements at a single measuring operation.

For such water quality checkers it is necessary to calibrate the respective measuring sensors prior to an actual measurement. Such a calibration has to be carried out according to procedures briefly described in the following.

For example, a solution of phthalate having a pH value of 4 as a standard solution for calibration use is put in a breaker as a calibration container. Measuring sensors other than a dissolved oxygen-measuring sensor can be calibrated by use of this standard solution as it is. However, also an operation of saturating the standard solution with air has to be carried out in order to use this standard solution also for calibrating said dissolved oxygen-measuring sensor. This saturation with air is carried out by bubbling, i.e. by introducing air into the standard solution.

After finishing of said bubbling, the respective measuring sensors are immersed in the standard solution for calibrating the respective measuring sensors under such condition. That is to say, the calibration of the respective measuring sensors is carried out on the basis of measured data from the standard solution derived via the respective measuring sensors. However, in order to obtain an accurate measured value in the case of a dissolved oxygen-measuring sensor, it is necessary to provide a flow rate of some extent or in excess of a certain extent to the standard solution brought into contact with a surface of the diaphragm of the sensor. To this end, the standard solution has to be stirred by use of a stirrer for achieving a reliable calibration of a conventional water quality checker.

Such kind of calibration is not without problems since, for example, not only the bubbling operation which is not required for other measuring sensors is mandatory for calibrating the dissolved oxygen-measuring sensor which needs a certain amount of time, but also a stirrer for giving a certain flow rate to the standard solution is required. Thus, the water quality checker becomes rather complicated with respect to its construction and large-sized.

It is an object of the invention to provide a water quality checker with simple construction equipped with a plurality of kinds of measuring sensors including a dissolved oxygen-measuring sensor and which can be calibrated easily.

According to a first aspect of the invention, a water quality checker comprising a sensor member body provided with a plurality of kinds of measuring sensors including at least a dissolved oxygen-measuring sensor arranged at one end portion thereof and a calibration container housing a standard solution for the calibration of said measuring sensors, is characterized in that said calibration sensor is provided with a partition wall portion for isolating said dissolved oxygen-measuring sensor arranged at the outside thereof, and in that said other measuring sensors intrude into the calibration container.

According to another aspect of the invention, a water quality checker comprising a sensor member body provided with a plurality of kinds of measuring sensors including at least a dissolved oxygen-measuring sensor arranged at one end portion thereof is characterized in that a sensing portion of said dissolved oxygen-measuring sensor is arranged at a position closer to said sensor member body as compared with sensing portions of said other measuring sensors.

The advantage of the constructional features according to the first aspect of the invention is that the dissolved oxygen-measuring sensor is kept outside of the calibration container and is isolated from the other measuring sensors immersing in said standard solution. Accordingly, all measuring sensors can simultaneously be calibrated by calibrating the dissolved oxygen-measuring sensor with air as a calibration standard gas requiring no bubbling and stirring, and the other measuring sensors can be calibrated by using the standard solution.

According to the constructional features of the second aspect of the invention, when the measuring sensors are immersed in the standard solution contained in said container for carrying out a calibration is that the sensing portion of the dissolved oxygen-measuring sensor is maintained at a height where it does not immerse into the standard solution, whereas all sensing portions of said other measuring sensors are held at a height where they immerse into the standard solution, in particular by regulating the height of the said sensor member body relatively to the momentaneous liquid level of said standard solution. Accordingly, also in this case, all measuring sensors can be simultaneously calibrated, i.e. by calibrating the dissolved oxygen-measuring sensor with air as said standard gas, whereas the other measuring sensors are calibrated by use of the standard solution.

The invention and further advantageous details thereof are described in the following with reference to specific examples and to the drawings wherein
- Fig. 1: is a perspective view of the external appearance of a water quality checker according to one preferred embodiment of the invention;
- Fig. 2: is a perspective view for explaining the procedures of calibrating various measuring sensors of said water quality checker;
- Fig. 3: is a longitudinal sectional view showing the positional relation between a sensor member and a calibration container during the calibration of said measuring sensors in said water quality checker;
- Fig. 4: is a perspective view of a calibration container in a water quality checker according to another preferred embodiment of the invention;
- Fig. 5: is a longitudinal sectional view of the calibration container of Fig. 4;
- Fig. 6: is a longitudinal sectional view similar to Fig. 3 for explaining the positional relation of sensor members and the calibration container; and
- Fig. 7: is a cross-sectional view seen in the direction of the arrows VII-VII in Fig. 6.

A preferred embodiment of the invention will be described in the following with reference to the said drawings.

A water quality checker according to a preferred embodiment comprises a sensor member 1, a checker body 3 electrically connected with said sensor member 1 through a cable 2, a protecting tube 4 detachably mounted on said sensor member 1 and a calibration container 5 for housing a standard solution for use during calibration.

The sensor member 1 comprises a sensor member body 6 having a circular lower end portion and a plurality of kinds of water quality sensors hanging down from a lower end surface of said sensor member body 6. In particular, those water quality sensors comprise a pH-measuring glass electrode 7 and a reference electrode 8, a conductivity cell 9 for measuring a conductivity value, a dissolved oxygen (DO) sensor 10 for measuring dissolved oxygen and a turbidity cell 11 for measuring a turbidity. In particular, said DO sensor 10 is arranged at a position closer to a circumferential lower end surface portion of said sensor member body 6.

The checker body 3 is a device taking over signals measured by the respective measuring sensors via said cable 2 for calibrating the measuring sensors and for operating measuring results with respect to the various items of water quality. Said operation is carried out automatically by means of a microcomputer in accordance with an instruction input after pressing an operating button 3a. The operation results are displayed on a display portion 3b.

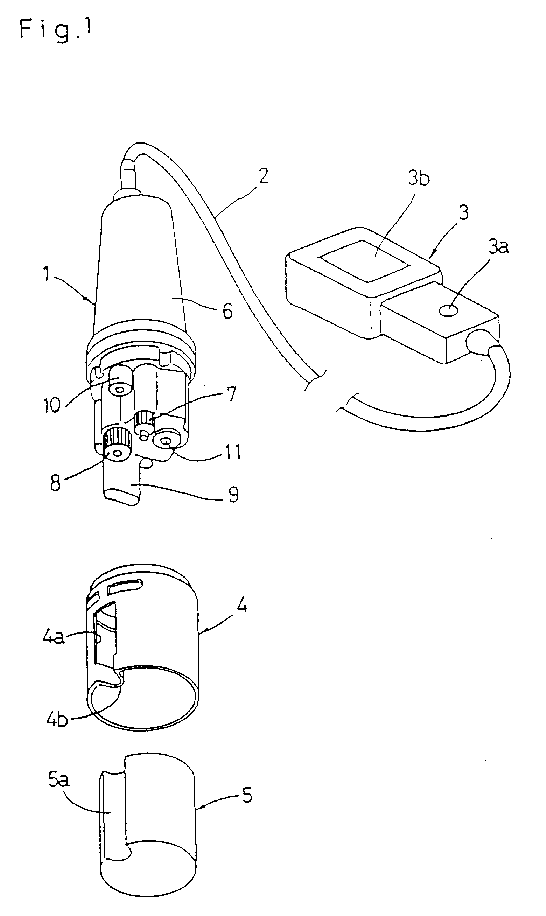

The protecting tube 4 is a member having an essentially cylindrical shape and surrounding the respective measuring sensors provided at the lower end portion of said sensor member body for protecting the measuring sensors against shocks and the like. The protecting tube 4 is usually slipped over, however, in Fig. 1 shown to be detached from the lower end portion of the sensor member body. The fixation between the protecting tube 4 and the sensor member body 6 is achieved by means of a bayonet-type fixation or the like. Said protecting tube 4 is provided with an opening 4a and a guide member 4b projecting inwardly at a circumferential position corresponding to the position where the DO sensor 10 is mounted. This inwardly projecting guide member 4b is of integral structure with the circumferential wall of said protecting tube 4.

The calibration container 5 is an essentially cylindrical container having an outside diameter fitting into the protecting tube 4 and provided with a partition wall 5a formed by sinking a part of the circumferential wall there of toward the inside for isolating only the DO sensor 10 which becomes arranged outside thereof. Accordingly, said inwardly projecting partition wall 5a separates said DO sensor 10 against the inside of the calibration container 5. Said partition wall portion 5a is also used as a groove for guidance by said guide member 4b of the protecting tube 4, when the measuring sensors are immersed in the standard solution within the calibration container 5 during calibration.

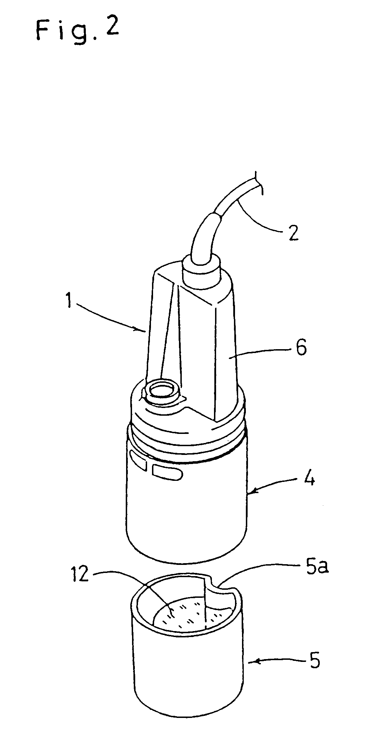

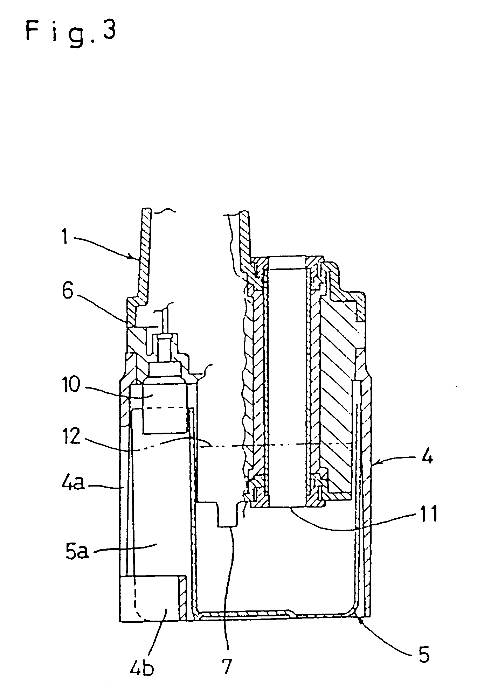

The perspective view of Fig. 2 shows the immersing of the measuring sensors of the sensor member 1 into the standard solution 12 within the calibration container 5 during calibration of the water quality checker. The longitudinal sectional view of Fig. 3 serves to explain in further detail a condition when the measuring sensors are immersed in the standard solution 12.

The operating procedures during calibration of the water quality checker will be described below with reference to Figs. 2 and 3.

The protecting tube 4 is mounted on the lower end portion of the sensor member body 6 and the sensor member 1 is descended into the calibration container 5 in which the standard solution 12 is contained, consisting of for example an aqueous solution of phthalate having a pH value of 4 (see Fig. 2). At this time, the position of the guide member 4b of the protecting tube 4 is arranged in adjustment of the partition wall 5a of the calibration container 5. As soon as the sensor member 1 is descended such that the calibration container 5 fits into the protecting tube 4 (see Fig. 3), the DO sensor 10 is positioned outside of the calibration container 5 and is such exposed to air. However, the other measuring sensors are immersed in the standard solution within the calibration container 5.

Accordingly, the DO sensor 10 detects the concentration of oxygen in an atmosphere, and the calibration with air as a standard gas can be carried out on the basis of the detected signals in the checker body 3. The calibration of the other measuring sensors is carried out in the same manner as in a conventional water quality checker, i.e. on the basis of signals derived from those measuring sensors as a result of the immersion into the standard solution 12. As can be readily understood from the above description, the calibration of the DO sensor 10 is carried out with air as a standard gas, however without requiring a bubbling treatment for the standard solution and without the need of a separate stirrer. Accordingly, the whole measuring apparatus is considerably simplified by an economically very reasonable and technically simple solution.

Since the protecting tube 4 is provided with the opening 4a formed at a circumferential position corresponding to the position of the DO sensor 10, a sufficient exposure to air of the DO sensor 10 without hindrance by the protecting tube 4 can be guaranteed. In addition, the protecting tube 4 functions also as a standing support member when the sensor 1 is put on a floor or the like.

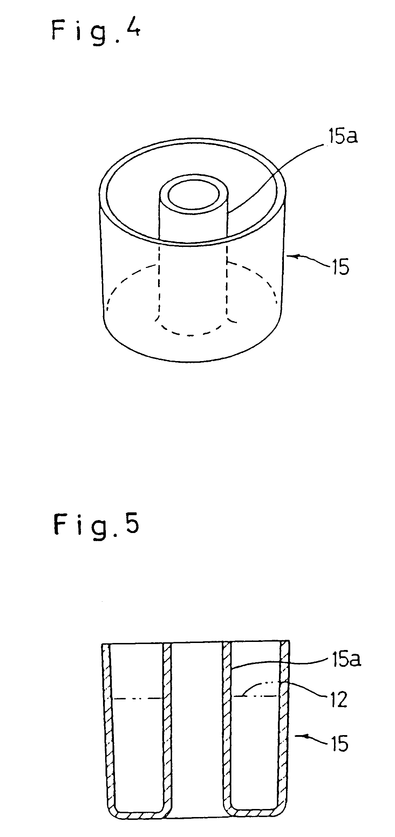

Fig. 4 shows a calibration container 15 for another preferred embodiment of a water quality checker according to the present invention. Fig. 5 depicts the longitudinal sectional view of the calibration container 15 of Fig. 4.

In this preferred embodiment, the calibration container 15 is provided with a cylindrical inner circumferential wall used as a partition wall portion 15a for isolating the DO sensor 10 against the interior of the calibration container 15. Corresponding to the cocentric arrangement of the partition wall portion 15a, the DO sensor 10 is installed in a central position of the lower end surface of the sensor member body 6, while the other measuring sensors are arranged around the DO sensor 10.

Also in this case, since merely the DO sensor 10 is exposed to air, whereas the other measuring sensors are immersed in the standard solution 12, the same calibration can be achieved as precedingly described in connection with the first preferred embodiment. In addition, although the calibration container 5 or 15, respectively, is provided with partition wall portions 5a and 15a, respectively, for isolating the DO sensor 10 to the outside thereof, in the case of the above-described preferred embodiments, it is also possible to held and maintain the sensing portion of the DO sensor 10 at a position higher than the liquid level of the standard solution 12 within the calibration container 5 and 15, respectively, during the calibration under the condition that the sensing portions of the other measuring sensors are immersing into the standard solution 12. This can be achieved by setting the position of the sensing portion of the DO sensor 10 at a height with respect to the lower end surface of the sensor member body 6 sufficiently higher than the relative positions of the sensing portions of the other measuring sensors. In other words, the sensing portion of the DO sensor 10 is arranged closer to the lower end surface of the sensor member body 6.

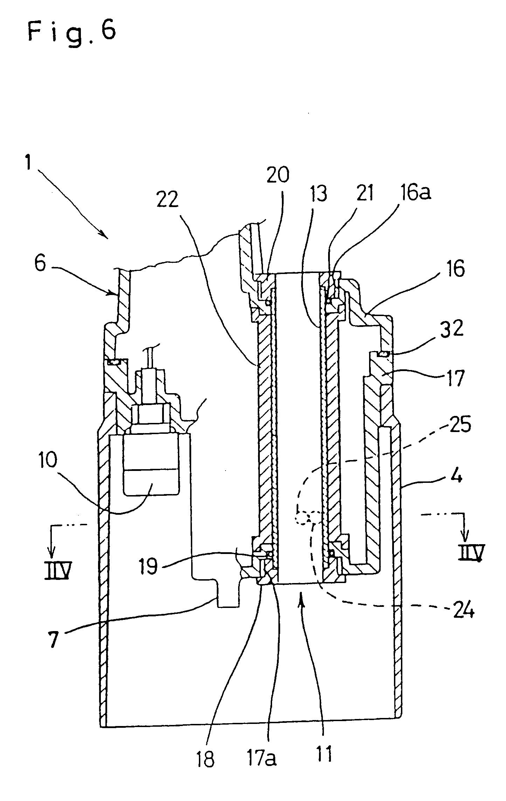

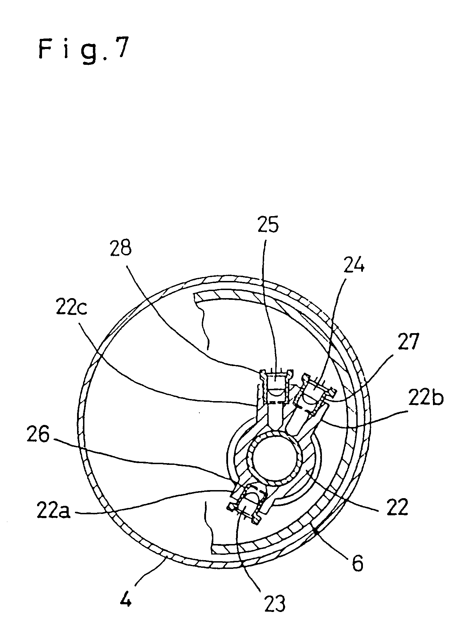

A preferred embodiment according to this type of construction is described in the following with reference to Figs. 6 and 7.

The sensor member body 6 comprises an upper frame 16 and a lower frame 19, which are joined by use of sealing O-ring 32. The sensor member body 6 is provided with a glass tube 13 having an upper end and a lower open end which is a transparent cylindrical tube forming a turbidity cell 11 vertically passing through said upper frame 16 to said lower frame 17.

Said glass tube 13 is fixed by means of a lower fixing screw 18 and a concaved sunk portion 17a of the lower frame 17 through which the glass tube 13 passes. An O-ring 13 is disposed between an upper end of said lower fixing screw 18 and said concave sunk portion 17a of the lower frame 17 to seal the lower end portion of said glass tube 13 and the lower frame 17.

In addition, the glass tube 13 is fixed by means of an upper fixing screw 20 screwed into a concave sunk portion 16a of the upper frame 16 through which the glass tube 13 passes.

An O-ring 21 is disposed between a lower end of the upper fixing screw 20 and said concave sunk portion 16a of the upper frame 16 to seal the upper end portion of the glass tube 13 against the upper frame 16.

In addition, the glass tube 13 is provided with a surrounding reinforcing tube member 22 and with lateral cylindrical members 22a, 22b formed at opposite positions on both sides thereof, and a separate lateral cylindrical member 22c formed at the position biased from said lateral cylindrical member 22b by an appointed turning angle so as to be opened toward the glass tube 13, respectively. Said lateral cylindrical member 22a is provided with a LED 23 as a light source. The lateral cylindrical member 22b is provided with a photodiode 24 as a transmitted light-receiving element, and said lateral cylindrical member 22c is equipped with a separate photodiode 25 as a scattered light-receiving element. Said LED 23, said photodiode 24 and said photodiode 25 are each provided with a cap 26, 27, 28, respectively, provided with a slit for limiting beams in order to avoid an irradiation of disturbing or unnecessary beams and for avoiding receipt of undesired beams at a specific end thereof, respectively.

In the turbidity-measuring cell 11 of the water quality checker, the glass tube 13 constructed as a window cell is provided for vertically passing through the sensor member body 6 so that, for example, in the case where the sensor member 1 is immersed in water when measuring the water quality of a river, not only water as the sample liquid enters the sensor member 1 from the lower end side of the glass tube 13 but also air within the glass tube 13 can disappear from the upper end side of the glass tube 13 so that a replacement of air within the sample liquid can easily be achieved without requiring specific grooves as in a conventional water quality checker.

Beams transmitting through the sample liquid contained in the glass tube 13 emitted by the LED 23 are received by the transmitted light-receiving photodiode 24, while beams scattered by the midway sample liquid are received by the scattered light-receiving photodiode 25, The turbidity of the sample liquid is measured as a ratio of a quantity of the transmitted light in relation to that of the scattered light in the same manner as with a turbidity-measuring sensor of a conventional water quality checker.

In this preferred embodiment, the glass tube 13 of the turbidity-measuring cell 11 is arranged vertically to the sensor member body 6 in the same manner as the other measuring sensors so that the turbidity-measuring cell 11 provides for a compact overall construction without limiting the arrangement of the other measuring sensors.

In addition, the inside of the glass tube 13 into which the sample liquid is filled and which forms the cell window of the turbidity-measuring cell 11, is smooth and a continuous circumferential surface without steps or notches so that bubbles, dirt and stain, do not or only hardly stick to the inside of the glass tube 13. This contributes considerably to not only avoid errors of measurement resulting from said dirt and stain and the like, but also facilitates the cleaning of the glass tube 13 whenever required.

In addition, if a sample liquid-supplying tube is connected to one end side of the glass tube 13 and a sample liquid-discharging tube is connected with the other end side of the glass tube 13, the device can also be used as a flow cell for continuous measurement of a sample liquid.

## Claims

1. A water quality checker comprising a sensor member body (6) provided with a plurality of kinds of measuring sensors (7, 8, 9, 10) including at least a dissolved oxygen-measuring sensor (10) at an end portion thereof and a calibration container (5; 15) housing a standard solution (12) used for calibrating said measuring sensors, **characterized in that** said calibration container (5; 15) is provided with a partition wall portion(5a; 15a) for isolating said dissolved oxygen-measuring sensor (10) to the outside of said calibration container (5; 15) and against said other measuring sensors (7, 8, 9), such that only said other measuring sensors (7, 8, 9) can intrude into the interior of said calibration container (5, 15).

2. A water quality checker comprising a sensor member body (6) provided with a plurality of measuring sensors (7, 8, 9, 10) including at least a dissolved oxygen-measuring sensor (10) at one end portion thereof, **characterized in that** the sensing portion of said dissolved-oxygen measuring sensor (10) is arranged at a position closer to a lower end surface of said sensor member body (6) as compared with the sensing portions of said other measuring sensors (7, 8, 9) so that during calibration of said sensors, the dissolved oxygen-measuring sensor (10) can be exposed to air as a standard calibration gas whereas the other measuring sensors (7, 8, 9) can be immersed into said standard solution (12) contained in said calibration container (5; 15).

## Patentansprüche

1. Wasserqualitätsprüfer mit einem Sensorteilkörper (6) mit mehreren Arten von Meßsensoren (7, 8, 9, 10) mit mindestens einem Meßsensor für gelösten Sauerstoff (10) in einem Endbereich desselben und mit einem Kalibrierbehälter (5; 15), der eine Standardlösung (12) enthält, die zum Kalibrieren der Meßsensoren verwendet wird, **dadurch gekennzeichnet,** daß der Kalibrierbehälter (5; 15) mit einem Trennwandbereich (5a; 15a) zum Trennen des Meßsensors für gelösten Sauerstoff (10) zur Außenseite des Kalibrierbehälters (5; 15) hin und gegen die anderen Meßsensoren (7, 8, 9) versehen ist, so daß nur die anderen Meßsensoren (7, 8, 9) in das Innere des Kalibrierbehälters (5, 15) eindringen können.

2. Wasserqualitätsprüfer mit einem Sensorteilkörper (6) mit mehreren Meßsensoren (7, 8, 9, 10) mit mindestens einem Meßsensor für gelösten Sauerstoff (10) in einem Endbereich desselben, **dadurch gekennzeichnet,** daß der Meßbereich des Meßsensors für gelösten Sauerstoff (10) an einer Position angeordnet ist, die näher an der unteren Endseite des Sensormeßkörpers (6) liegt als die Meßbereiche der anderen Meßsensoren (7, 8, 9), so daß der Meßsensor für gelösten Sauerstoff (10) während der Kalibrierung der Sensoren Luft als Standardkalibriergas ausgesetzt sein kann, wohingegen die anderen Meßsensoren (7, 8, 9) in die Standardlösung (12) eingetaucht sein können, die im Kalibrierbehälter (5; 15) enthalten ist.

## Revendications

1. Dispositif de contrôle de la qualité de l'eau, comportant un corps (6) d'élément de détection pourvu à une extrémité de plusieurs types de sondes de mesure (7, 8, 9, 10) comprenant au moins une sonde (10) de mesure d'oxygène dissous et un récipient d'étalonnage (5; 15) contenant une solution standard (12) servant à étalonner lesdites sondes de mesure, **caractérisé en ce que** ledit récipient d'étalon-nage (5; 15) est pourvu d'une partie formant cloison (5a; 15a) pour isoler ladite sonde (10) de mesure d'oxygène dissous par rapport à l'extérieur dudit récipient d'étalon-nage (5; 15) et vis à vis lesdites autres sondes de mesure (7, 8, 9), de telle façon que seules lesdites autres sondes de mesure (7, 8, 9) puissent pénétrer à l'intérieur dudit récipient d'étalonnage (5, 15).

2. Dispositif de contrôle de la qualité de l'eau, comportant un corps (6) d'élément de détection pourvu à une extrémité de plusieurs sondes de mesure (7, 8, 9 10) comprenant au moins une sonde (10) de mesure d'oxygène dissous, **caractérisé en ce que** la partie détectrice de ladite sonde (10) de mesure d'oxygène dissous est disposée plus près de la surface d'extrémité inférieure dudit corps (6) d'élément de détection que les parties de détection desdites autres sondes de mesure (7; 8, 9) de façon que, pendant l'étalonnage desdites sondes, la sonde (10) de mesure d'oxygène dissous puisse être au contact d'air constituant un gaz d'étalonnage de référence, tandis que les autres sondes de mesure (7, 8, 9) peuvent être plongées dans ladite solution standard (12) contenue dans ledit récipient d'étalonnage (5; 15).
